# FASCICULE DE BREVET EUROPEEN

(11) **EP 4 349 257 B1**
(45) Date de publication et mention de la délivrance du brevet: **23.07.2025**
(21) Numéro de dépôt: 23200144.6
(22) Date de dépôt: 27.09.2023
(51) Int. Cl.: A61B 5/20, A61B 5/00, G01S 13/32, G01S 7/41

(54) **DISPOSITIF RADAR POUR TOILETTES**
RADARVORRICHTUNG FÜR TOILETTEN
RADAR DEVICE FOR TOILET

(30) Priorité: 03.10.2022 FR 2210069
(43) Date de publication de la demande: 10.04.2024
(73) Titulaire: Withings, 92130 Issy-Les-Moulineaux (FR)
(72) Inventeur: Koczan, Xavier, Issy-les-Moulineaux (FR)
(74) Mandataire: Withings IP

(56) Documents cités:
- WO-A1-2021/055681
- WO-A1-2021/175944
- JP-A- 2009 036 585
- US-A1- 2020 268 303

## Description

La présente description concerne les dispositifs et les méthodes d'identification d'un utilisateur de toilettes. Une telle identification trouve notamment application dans le cadre de tests d'analyse d'urine, où il peut être souhaité de pouvoir identifier l'auteur de la miction pour attribuer les résultats de l'analyse d'urine à la bonne personne, ou déclencher une mesure d'analyse d'urine.

L'analyse d'urine est aujourd'hui essentiellement effectuée en laboratoire spécialisé ou bien à la maison de façon assez rudimentaire. Dans les deux cas, l'intervention active d'une personne est requise, que ce soit un technicien de laboratoire ou l'utilisateur lui-même, afin de mettre en oeuvre le prélèvement d'urine : l'identification de l'utilisateur ne pose aucune difficulté technique.

La présente description concerne également l'analyse d'un jet d'urine par des méthodes non invasives.

### Etat de la technique

Les évolutions techniques sont en train de rendre possible les tests automatisés à la maison. Par automatisé, il est signifié que les actions de l'utilisateur sont réduites à un minimum. Par exemple, les documents WO2021/175909, WO2021/175944 et FR2101762 décrivent un dispositif d'urine autonome à positionner dans les toilettes au domicile de l'utilisateur. Une des problématiques liées à ce genre de dispositifs, qui prélèvent automatiquement de l'urine et l'analysent, est l'attribution de la mesure à un utilisateur précis en train d'uriner parmi une pluralité d'utilisateurs potentiels. Il existe donc un besoin de pouvoir identifier l'utilisateur. Les documents précités décrivent quelques pistes, comme une interaction avec un bouton ou bien une reconnaissance Bluetooth. Le document « A moun-table toilet system for personalized health monitoring via the analysis of excreta", Park et al, in Nature Biomedical engineering (DOI: 10.1038/s41551-020-0562-5), propose plusieurs moyens, comme un module d'identification d'empreintes digitales ou d'empreinte anale (« *analprint* »)*.* Le document WO2021/055881 décrit des dispositifs, systèmes et méthodes relatifs à des toilettes pour le suivi automatique de miction ou de défécation d'un utilisateur pour améliorer le diagnostic et le traitement de maladies ou autres. Il utilise des cellules de charge pour identifier un utilisateur (paragraphe [0064]) ou la possibilité de détecter un *wearable* de l'utiisateur, tel qu'un bracelet RFID (paragraphe [0066]). Le document JP2009036585 décrit un dispositif de mesure de température de l'urine. L'unité de contrôle détermine si un bouton de sélection individuel a été pressé pour identifier l'utilisateur du dispositif (paragraphes [0051] et [0132]).

Ces techniques existantes présentent des inconvénients : nécessité d'avoir son téléphone, nécessité d'installer un bouton, hygiène, invasion de vie privée, interaction nécessaire, etc.

Il est souhaitable de bénéficier d'un système d'identification ne présentant pas les inconvénients précités.

### Résumé de l'invention

La présente description vise à proposer une méthode et les dispositifs ou systèmes associés ne présentant au moins pas une des difficultés précitées. Plus spécifiquement, la présente description propose d'utiliser un radar pour identifier un utilisateur de toilettes. Notamment, les analyses d'urine peuvent faire partie des analyses sexuées, en ce sens où l'analyse indifférente de l'urine d'un homme ou d'une femme n'est pas forcément pertinente. Par conséquent, l'identification peut, dans le cadre de la description, signifier identifier le sexe de l'utilisateur.

L'invention est définie dans les revendications.

Dans un mode de réalisation, la description présente une méthode de mesure relative à un jet d'urine d'un utilisateur en cours de miction pour identifier un utilisateur de toilettes la méthode de mesure utilisant un capteur radar et comprenant au moins les étapes suivantes :
l'émission par le capteur radar d'au moins un signal radar, en direction du jet d'urine,
la réception par le capteur radar d'un signal radar réfléchi, le signal radar reçu comprenant des réflexions du signal émis, les réflexions étant provoquées par au moins le jet d'urine,
le traitement du signal radar reçu pour déterminer au moins une propriété relative au jet d'urine.

En particulier, le capteur radar est installé dans les toilettes, sur une paroi d'une cuvette.

La propriété relative au jet d'urine peut comprendre au moins une distance d'intérêt entre le capteur radar et le jet d'urine, par exemple la distance entre l'origine du jet d'urine et le capteur radar.

Dans un mode de réalisation, ladite distance d'intérêt est obtenue par :
- l'identification d'une vitesse radiale d'intérêt liée à la vitesse radiale maximale (Vmax) d'un front d'urine,
- l'obtention de la distance radiale (Rmax) correspondant à cette vitesse d'intérêt, ladite distance radiale (Rmax) correspondant à la distance d'intérêt.

Dans un mode de réalisation, la propriété relative au jet d'urine comprend un niveau de dispersion (NPix) du jet d'urine. En particulier, ledit niveau de dispersion est obtenu par le calcul d'un niveau de réflexion des signaux radars réfléchis.

Dans un mode de réalisation, la propriété du jet d'urine comprend au moins une vitesse d'intérêt d'un front d'urine. La vitesse d'intérêt du jet d'urine peut comprendre la vitesse mesurée maximale (Vmax) du jet d'urine.

La méthode de mesure comprend en outre l'attribution du jet d'urine à un profil d'utilisateur parmi une pluralité de profils d'utilisateur à l'aide de l'au moins une propriété du jet d'urine. L'attribution peut comprendre une attribution entre un profil d'utilisateur associé à un homme et un profil d'utilisateur associé à une femme.

Dans un mode de réalisation, la classification se fait à l'aide de la vitesse mesurée maximale et du niveau de dispersion et/ou se fait en combinant plusieurs propriétés du jet d'urine, dont notamment la vitesse mesurée maximale du jet d'urine.

Dans un mode de réalisation, l'attribution se fait par classification sur la base d'au moins une propriété relative au jet d'urine et d'une fonction de classification. La fonction de classification peut être obtenue au préalable à partir d'un ensemble de données (notamment pour l'apprentissage du classificateur).

Dans un mode de réalisation, le capteur radar fonctionne par frame, chaque frame étant générée par une pluralité de chirps, et les étapes d'émission et de réception étant mises en oeuvre pour chaque chirp.

En particulier, le capteur radar est un capteur radar de type « *Frequency Modulated Continuous Wave* », FMCW, ou le signal radar est FMCW. Les fréquences du capteur radar peuvent varier entre 58 GHz et 63 GHz.

Le traitement du signal peut comprendre un calcul d'au moins une réponse « range-doppler », par exemple une carte « distance-doppler ».

Dans un mode de réalisation, la méthode de mesure comprend une étape préalable, à l'aide d'un détecteur d'urine, de détermination de la présence d'un jet d'urine.

La description présente aussi un dispositif radar comprenant un capteur radar apte à mettre en œuvre une méthode telle que décrite précisément.

La description présente aussi un dispositif radar comprenant :
- un boîtier, apte à être positionné sur une paroi interne d'une cuvette de toilette,
- un capteur radar, logé dans le boitier, et apte à émettre des ondes radar en direction de l'ouverture de la cuvette de toilette,

Le capteur radar peut être un capteur radar de type « *Frequency Modulated Continuous Wave* »*,* FMCW.

Le dispositif radar peut comprendre un détecteur d'urine apte à détecter un jet d'urine, le détecteur d'urine étant configuré pour activer le capteur radar en réponse à une détection du jet d'urine.

La description présente aussi un dispositif d'analyse d'urine, comprenant :
- un dispositif radar tel que décrit précédemment,
- un orifice de collecte sur le boitier pour recevoir de l'urine,
- un ensemble de test destiné à analyser l'urine reçue.

Le dispositif radar peut comprenant une batterie pour alimenter en énergie le ou les capteur radar.

En particulier, le boitier peut être étanche.

Enfin, la description présente un programme d'ordinateur comprenant des instructions aptes à mettre en oeuvre une méthode telle que décrite auparavant lorsque les instructions sont exécutées par un processeur. Ce programme d'ordinateur peut être mis en oeuvre par le dispositif radar tel que décrit précédemment.

En particulier, une contrainte forte à l'identification est liée à la gestion de l'énergie du dispositif d'analyse d'urine auquel le radar peut être intégré : le capteur radar doit être activé aussi peu que possible. Par conséquent, envoyer des ondes radar prospectives demeure peu envisageable, puisque pour détecter le mouvement debout assis, il faut activer le capteur radar avant que l'utilisateur n'apparaisse dans le champ de vision du radar. Pour procéder de la sorte, soit le capteur radar envoie des ondes radar prospectives, soit le capteur radar est informé de l'arrivée d'un utilisateur (mais cette solution complexifie encore la réalisation). La solution proposée ici permet un fonctionnement optimisé sur une batterie.

### Présentation des figures

Les figures suivantes permettent de favoriser la compréhension de l'invention :
- [FIG. 1] : la figure 1 présente schématiquement une vue en coupe de toilettes équipées d'un dispositif radar selon un mode de réalisation de la description,
- [FIG. 2] : la figure 2 présente une vue plus détaillée d'un boitier d'un dispositif radar associé à un dispositif d'analyse d'urine, selon un mode de réalisation de la description,
- [FIG. 3] : la figure 3 présente schématiquement une vue des composants d'un dispositif radar selon un mode de réalisation de la description, ainsi que son écosystème,
- [FIG. 4] : la figure 4 présente schématiquement une vue plus détaillée d'un boitier d'un dispositif radar associé à un dispositif d'analyse d'urine, selon un mode de réalisation de la description,
- [FIG. 5] : la figure 5 présente plusieurs situations représentant l'origine du jet d'urine et sa direction, par rapport au FoV du capteur radar,
- [FIG. 6] : la figure 6 présente deux cartes « distance-doppler », pour un homme et une femme, selon un mode de réalisation de la description,
- [FIG. 7] : la figure 7 présente une représentation tridimensionnelle de jets d'urine à l'aide de trois paramètres relatifs au jet d'urine,
- [FIG. 8] : la figure 8 présente des graphes bidimensionnelles représentations des projections de la figure 7,
- [FIG. 9] : la figure 9 présente un diagramme représentant un procédé de mesure selon un mode de réalisation de l'invention,
- [FIG. 10] : la figure 10 présente un diagramme représentant un procédé de mesure selon un mode de réalisation de l'invention, avec détection préalable d'urine,
- [FIG. 11] : la figure 11 présente une vue schématique du placement du capteur radar dans le boitier et/ou dans les toilettes, et
- [FIG. 12] : la figure 12 présente une vue schématique d'un dispositif d'analyse d'urine intégrant le dispositif radar, selon un mode de réalisation de la description.

### Description détaillée

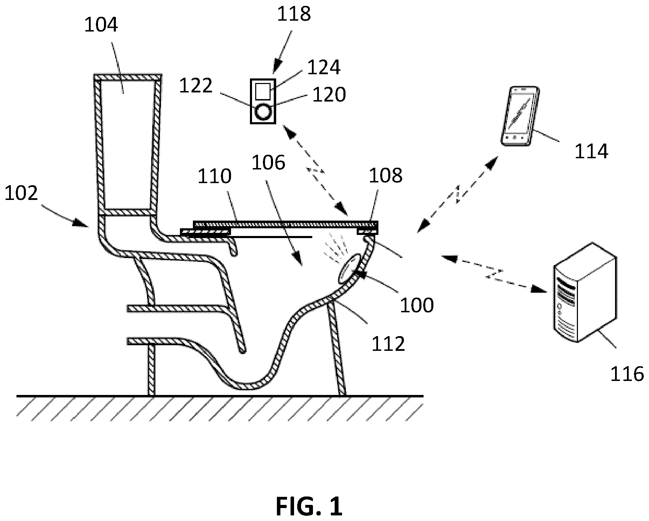

La figure 1 illustre schématiquement un dispositif radar 100 monté sur des toilettes 102. De manière connue, les toilettes 102 comportent un réservoir d'eau 104, une cuvette 106, un siège 108 et un couvercle 110. Le dispositif d'identification 100 peut être agencé sur une paroi interne 112 de la cuvette 106 des toilettes. Avantageusement, le dispositif radar 100 est entièrement reçu dans la cuvette des toilettes, ce qui lui permet d'être discret.

Dans un mode de réalisation, le dispositif radar 100 peut être positionné dans les toilettes de sorte à être sur la trajectoire d'un jet d'urine secrété par un utilisateur en cours de miction, notamment lorsqu'un utilisateur urine en position assise dans les toilettes. La position du dispositif d'analyse d'urine dans les toilettes est alors adaptée pour tout type d'utilisateur, de sexe masculin ou féminin, quel que soit son âge. L'utilisateur peut alors uriner dans les toilettes sans se préoccuper de la position du dispositif d'analyse d'urine.

Le positionnement du dispositif radar 100 lui permet également d'être positionné sur la trajectoire d'une chasse d'eau provenant du réservoir 104. Le dispositif d'identification 100 peut ainsi être rincé lors de l'actionnement de la chasse d'eau.

Une attache peut être prévue pour maintenir le dispositif radar 100 sur la paroi interne de la cuvette : ventouse, aimant (avec support collé sur la paroi), crochet atteignant le rebord de la cuvette, etc.

Le dispositif radar 100 peut communiquer avec un terminal mobile 114 (type smartphone) et/ou un serveur externe 116. Dans un mode de réalisation, le dispositif radar 100 communique avec le terminal mobile 114 (par exemple directement via Bluetooth comme du Bluetooth Low Energy) et le terminal mobile 114 communique avec le serveur 116 (via une connexion cellulaire ou WiFi). Dans un autre mode de réalisation, le dispositif radar 100 peut communiquer directement avec le serveur 116 par un réseau cellulaire.

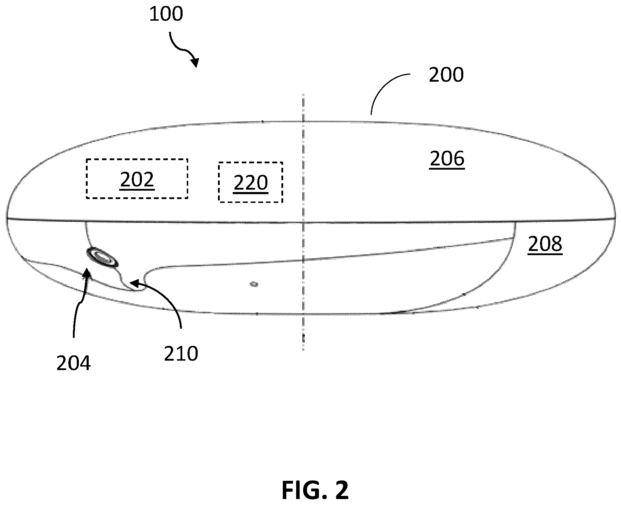

En référence à la figure 2, le dispositif radar 100 peut comprendre un boîtier 200 à l'intérieur duquel un capteur radar 202 est positionné (représenté schématiquement en pointillé). Le boîtier 200 est dimensionné pour pouvoir être positionné dans la cuvette 106 des toilettes 102. Du fait de son positionnement dans une région exposée à différents liquides ou solides, le boîtier 200 est étanche. Dans un mode de réalisation, le boitier 200 comprend un orifice de collecte 204, apte à recevoir de l'urine ruisselant sur le boîtier 200. Dans ce mode de réalisation, le dispositif radar 100 fait partie d'un dispositif d'analyse d'urine qui comprend notamment le boîter 200. Le boîtier 200 peut comprendre une coque avant 206 et une coque arrière 208, assemblables et démontables pour pouvoir accéder à l'intérieur du boîtier 200. Le dispositif d'analyse d'urine a été décrit dans les documents WO2021/175909, WO2021/175944 (numéros de publication), FR2109383, FR2109384, FR2109391, et FR2109392 (numéros de dépôt).

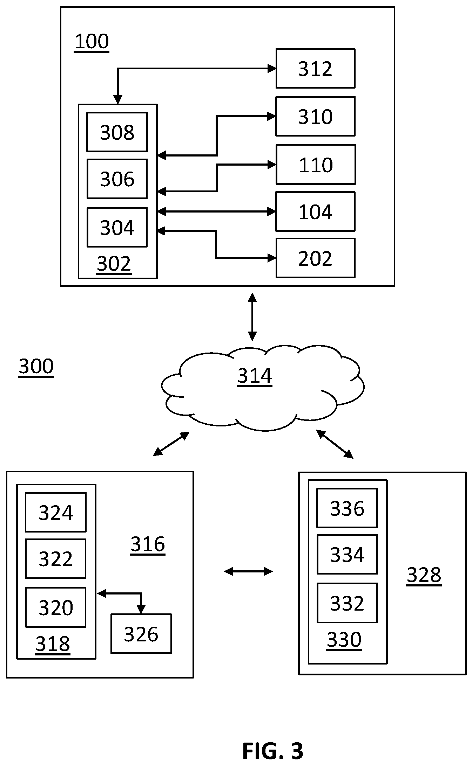

La figure 3 représente, dans un schéma 300, les composants que peut comprendre le dispositif radar 100, et l'écosystème général. Le dispositif radar 100 comprend une circuiterie de contrôle 302 avec un processeur 304, une mémoire 306 et une interface I/O (entrée/sortie, « *input*/*output* ») 308 configurée pour envoyer et recevoir des données depuis la circuiterie de contrôle 302. Un module de communication 310 peut être prévu pour échanger des données avec un terminal externe (par exemple un smartphone). Le module de communication 310 peut être un module sans fil, de type Wi-Fi, Bluetooth, Bluetooth Low Emission, etc. La circuiterie de contrôle 302 peut notamment échanger avec le capteur radar 202 pour envoyer des instructions d'acquisition et recevoir des données radar à traiter.

Le dispositif radar 100 peut comprendre une batterie 312 qui alimente les composants en énergie.

La mémoire 306 peut stocker des instructions, qui, lorsqu'exécutées par le processeur 304, mettent en œuvre la ou les méthodes de la présente description. Les méthodes sont préférablement effectuées en local, par le processeur 304 du dispositif radar 100. Cela permet un retour à l'utilisateur sans connexion nécessaire avec notamment le terminal externe (« *smartphone* »)*.*

Le dispositif radar 100 peut communiquer, à l'aide du module de communication 310 et à l'aide d'un réseau de communication 314, avec un terminal mobile externe 316, de type terminal mobile (« *smartphone* »). Le terminal mobile 316 comprend une circuiterie de contrôle 318 avec un processeur 320, une mémoire 322 et une interface I/O 324 configurée pour envoyer et recevoir des données depuis la circuiterie de contrôle 302. Le terminal externe 316 comprend en outre un interface utilisateur 326 pour interagir avec l'utilisateur. Le processeur 320 et la mémoire 322 peuvent mettre en œuvre une application qui permet au terminal externe 316 de communiquer avec le dispositif de mesure 100. L'interface utilisateur 326 peut notamment afficher des informations à l'utilisateur.

Le dispositif radar 100 peut aussi communiquer avec un serveur 328, soit directement via le réseau de communication 314 soit via le terminal externe 316. Le serveur 328 comprend une circuiterie de contrôle 330 avec un processeur 332, une mémoire 334 et une interface I/O 336 configurée pour envoyer et recevoir des données depuis la circuiterie de contrôle 302. Le serveur 328 peut stocker les mesures effectuées par le dispositif radar 100(architecture nuage « cloud »). Le serveur 328 peut aussi effectuer du traitement de données.

Le réseau de communication 314 peut être hétérogène : sans-fil courte portée (Bluetooth, Wi-Fi, etc.), sans-fil longueur portée (cellulaire, etc.), câblé (Ethernet, etc.).

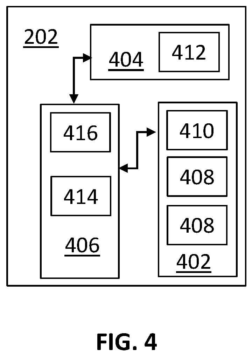

La figure 4 décrit plus en détail un exemple de capteur radar 202. Le capteur radar 202 comprend un émetteur 402, un récepteur 404 et une circuiterie de contrôle 406. L'émetteur 402 comprend au moins une antenne de transmission Tx 408 et un générateur d'onde 410. Le récepteur 404 comprend au moins une antenne de réception Rx 412. La circuiterie de contrôle 406 comprend notamment un processeur 414 et une mémoire 416, pour piloter l'émetteur 402 et traiter les signaux reçus par le récepteur 404. La circuiterie de contrôle 406 du capteur radar 202 peut être intégrée ou partiellement intégrée à la circuiterie de contrôle 302 du dispositif radar 100. On parlera par la suite de « circuiterie de contrôle 302, 406 » pour désigner l'une et/ou l'autre. Le capteur radar 202 utilise notamment l'effet Doppler-Fizeau généré par un objet en mouvement pour obtenir notamment la vitesse dudit objet et/ou la distance entre ledit objet et le capteur radar 202. La vitesse est dite radiale, car il s'agit uniquement de la composante de vitesse projetée sur un axe reliant ledit objet et le capteur radar 202. De la même façon, on parlera de distance radiale, car il s'agit de la distance le long de cet axe.

Le générateur d'onde 410 et l'antenne Tx génèrent des ondes électromagnétiques, émises en direction d'un champ de vue FoV (« *Field of view* »). Ces ondes électromagnétiques sont partiellement réfléchies par les obstacles qu'elles rencontrent et créent un écho qui est reçu par l'antenne Rx 404. La circuiterie de contrôle 302, 406 traite les échos pour générer des données radar. La circuiterie de contrôle 302, 406 peut convertir les signaux analogiques générés par le générateur d'onde 310 et reçus par l'antenne Rx en signaux numériques. Des filtres, amplificateurs, etc. sont typiquement prévus dans le capteur radar 202.

Le capteur radar 202 peut être compact, de l'ordre de quelques centimètres, voire moins de 1 cm. Par exemple, le capteur radar 202 peut être contenu dans un cube de 1 cm x 1cm x 1 cm.

Le champ de vue FoV est typiquement un angle solide, qui couvre un volume de l'espace à partir du capteur radar 202. Le FoV est généralement défini par deux angles d'ouverture. L'axe de symétrie de chaque angle est appelé axe du radar.

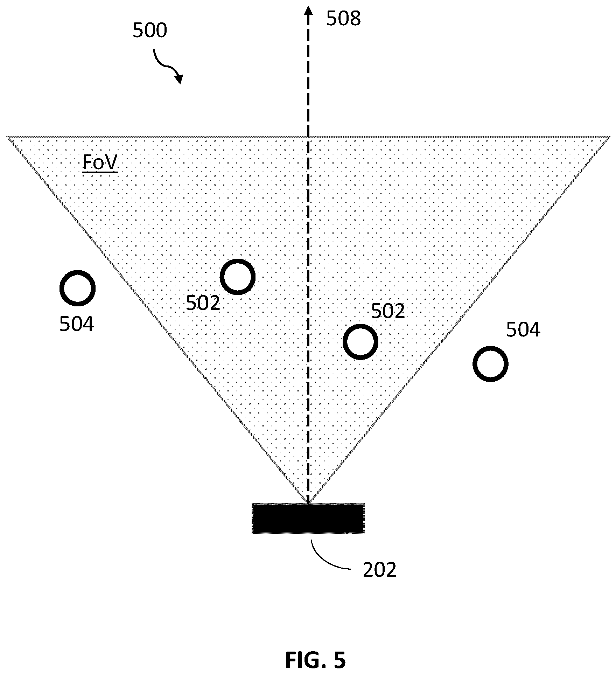

En référence à la figure 5, le capteur radar 202 peut être un radar à onde continue modulée en fréquence, dit FMCW (« *Frequency Modulated Continuous Wave* »)*,* ce qui signifie que le capteur radar émet un signal modulé en fréquence (« *chirp* » selon la terminologie consacrée). Formulé autrement, durant l'impulsion, d'une durée T, la fréquence du chirp émis varie sur une plage. Plusieurs modulations sont possibles : modulation en dents-de-scie, modulation triangulaire, modulation par déplacement de fréquence, modulation en escalier, etc. Le capteur radar peut être un radar à bande ultra-large, dit UWB (« Ultra Wide Band ») peut aussi convenir. Le radar UWB émet de motifs d'ondes de quelques nanosecondes qui sont répétés. L'étude des retards permet de déterminer des distances et l'études des variations de retard permet de détermine des vitesses.

Le capteur radar 202 peut émettre une succession de chirps, la succession étant appelée « frame » (traduisible par « fenêtre » en français mais le terme anglais est couramment utilisé). Dans un mode de réalisation, une frame comprend entre 16 et 256 chirps, voire entre 32 et 64 chirps (par exemple 128 chirps). Plus spécifiquement, une frame peut se décomposer comme suit : N.(PRT)=N.(t_chirp + t_pause), où le PRT est le temps de répétition de l'impulsion (« *pulse repetition time* »), où t_chirp est le temps d'un chirp,t_pause est le temps de pause avant le chirp suivant et N est le nombre de chirps. Le PRT peut durer entre 300µs et 500µs. La pause peut faire 100µs. Une frame peut ainsi durer quelques millisecondes.

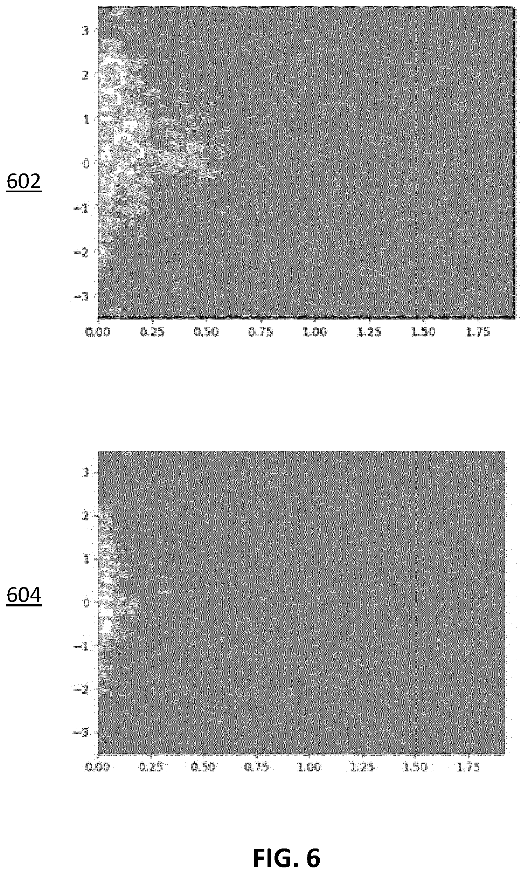

La modulation en fréquence permet de créer, après mixage des signaux émis et reçu, filtrage, etc., un signal, dit signal de fréquence intermédiaire (« *Intermediate Frequency Signal* ») dont les fréquences sont proportionnelles à la distance des objets à l'origine des échos. Une transformée de Fourier appliquée à ce signal de fréquence intermédiaire permet de mettre en avant les fréquences et les distances associées. En analysant les variations de phases des transformées de Fourier sur des chirps successifs, on peut mettre en évidence les fréquence Doppler, qui sont liées à la vitesse de l'objet. Le capteur radar 202 peut obtenir, pour chaque objet, la vitesse et la distance. En particulier, le capteur radar 202 peut générer une carte « Distance-Doppler » (« Range-Doppler » selon la terminologie usuelle), qui représente la distance (sur les figures en abscisse, en m) et la vitesse d'un objet en mouvement dans le champ de vue FoV (sur les figures en ordonnée, en m/s), comme représentée sur la figure 6. Pour chaque frame, une carte « Distance-Doppler » peut être calculée.

Dans le cadre des frames, l'obtention de la carte « Distance-Doppler » se fait à l'aide de FFT (« *fast fourier transform* ») et de leur évolution entre chirps successifs. Les cartes « Distance-Doppler » sont connues et ne seront pas décrites plus en détail.

A l'aide notamment des chirps, le capteur radar 202 peut aussi calculer une distance entre lui-même et un objet mouvant.

Une frame peut durer 100ms. Par conséquent, vingt frames successifs prennent 2s. Plus généralement, dans le cas d'un radar FMCW, un chirp peut durer entre 100 et 200 ms.

Dans les exemples illustrés de la description, le capteur radar 202 est un radar FMCW Infineon BGT60TR13C dont la fréquence peut varier entre 58 GHz et 63.5 GHz au cours d'un chirp. Cet intervalle autorise une bande passante de plus de 5 GHz ce qui assure une précision suffisante pour l'usage présenté dans la description. D'autres valeurs de fréquence peuvent être utilisées, en particulier autour des valeurs décrites. Ce capteur radar comprend trois antennes Rx et une antenne Tx. Dans l'exemple illustré, le chirp comprend une modulation de fréquence en dents de scie.

Pour favoriser la qualité du signal et pour mieux capteur les réflexions des ondes dans les toilettes, le dispositif radar 100 est disposé dans la cuvette de façon à ce que le champ de vue FoV du capteur radar 202 soit orienté en direction de l'ouverture de la cuvette, ce qui signifie qu'un utilisateur assis sur le siège 108 est dans la couverture radar et en particulier son postérieur, ses organes génitaux et l'orifice de sortie de l'urètre, qui est l'origine du jet d'urine de l'utilisateur.

Du fait des différences anatomiques entre un homme et une femme, la position de l'origine du jet d'urine n'est pas la même lorsque l'utilisateur est assis sur le siège 108. De plus, le jet d'urine est différent entre un homme et une femme pour différentes raisons morphologiques (forme de l'urètre, pression, débit, etc.).

Dans un mode de réalisation, le dispositif radar 100, au moyen d'un ou plusieurs frames, peut permettre d'identifier des propriétés du jet d'urine, ces propriétés permettant notamment de classifier le jet d'urine comme appartenant à un utilisateur donné. La classification d'un utilisateur peut être a *minima* une classification par discrimination entre homme et femme. Dans un ménage bigame hétérosexuel où les seuls utilisateurs du dispositif radar 100 sont l'homme et la femme, cette discrimination anatomique fondée sur le sexe permet d'identifier l'utilisateur du dispositif radar 100.

A cause de différences anatomiques entre un homme et une femme, des variations propres au placement de chaque dispositif radar 100 et des aux formes des cuvettes de toilettes, plusieurs situations peuvent survenir lors d'une miction assise. Ces situations sont représentées sur la figure 5, qui illustrent quatre configuration 500, 502, 504, 506 en deux dimensions, avec le capteur radar 202, l'axe du radar 508, le champ de vue FoV et la position de l'origine (le cercle) du jet d'urine et la direction globale (la flèche en pointillé partant du rond) du jet d'urine.

Dans un premier cas 502, l'origine du jet d'urine est située dans le champ de vue FoV du capteur radar 202 : dans ce cas, le capteur radar 202 perçoit des réflexions directes de haute intensité ainsi que les réflexions multiples issues des rebonds des ondes sur la cuvette de moindre vitesse et intensité.

Dans un deuxième cas 504, l'origine du jet d'urine situé hors du champ de vue FoV du capteur radar 202 : dans ce cas, le capteur radar 202 ne perçoit plus les réflexions directes. Le signal est alors formé que des seules réflexions multiples de faible intensité et faible vitesse à des distances plus grandes. De plus, du fait de la disposition du capteur radar 202, si l'origine du jet d'urine est hors du champ de vue FoV, cela implique que le jet d'urine sera probablement de plus faible longueur.

Dans le premier cas 502, la distance entre l'origine du jet et le dispositif radar 100 peut être déterminée pour identifier l'utilisateur. Dans le deuxième cas 504, le niveau de réflexion peut être déterminé pour identifier l'utilisateur, comme cela sera expliqué plus en détail par la suite. Pour des raisons anatomiques, le deuxième cas 504 se produit généralement pour des hommes.

Dans un mode de réalisation, le capteur radar 202 émet un chirp et reçoit un signal réfléchi. Ce signal réfléchi est ensuite traité par un processeur (soit la circuiterie de contrôle 406 du capteur radar 202, soit la circuiterie de contrôle 302 du dispositif radar 100) pour générer notamment, après émission d'une pluralité de chirps et réception des signaux réfléchis (i.e., une image), une carte « distance-doppler ». La figure 6 illustre deux cartes 602, 604 « distance-doppler » : la carte 602 illustre les résultats pour un utilisateur femme et la carte 604 illustre les résultats pour un utilisateur homme. A partir de ces cartes, plusieurs propriétés relatives au jet d'urine peuvent être obtenues : la distance radiale, la vitesse radiale et un niveau de dispersion.

Une carte « distance-doppler » peut être générée à partir d'une seule frame (i.e. calculée à partir d'une pluralité de chirps).

Comme indiqué précédemment une carte « distance-doppler » représente l'intensité du signal réfléchi (qui est lié au nombre d'objets en mouvement) en fonction de la distance radiale entre l'objet en mouvement et le dispositif radar 100 et en fonction de la vitesse radiale de cet objet. Une vitesse négative représente un objet qui s'éloigne et une vitesse positive représente un objet qui se rapproche.

Dans la présente description, l'objet en mouvement est un front d'urine. Par front d'urine, il est signifié l'interface air-urine d'un volume d'urine (par exemple une goutte ou une portion de jet) Un jet d'urine comprend typiquement une pluralité de fronts d'urine successifs. La carte 602 illustre ainsi des signaux radar qui correspondent à des fronts d'urine à une distance supérieure à ceux observés sur la carte 604, ce qui correspond à une différence anatomique : l'orifice du jet d'urine chez une femme assise sur les toilettes sera plus éloigné du capteur radar 202 que l'orifice du jet d'urine chez un homme assis sur les toilettes.

La carte 602 illustre aussi des signaux radar qui correspondent à des fronts d'urine ayant une vitesse radiale plus importante, ce qui correspond à nouveau à une différence anatomique : la vitesse d'éjection de l'urine est plus importante chez les femmes, à cause des voies d'évacuation masculines plus longues qui génèrent des pertes de charge. La carte 602 illustre des signaux radar plus dispersés que ceux observés sur la carte 604, ce qui correspondent à une pluralité d'objets (le jet d'urine et ses réflexions) plus éclatées : du fait de la distance plus importante chez les femmes que chez les hommes, du fait de la nature de l'urètre, la probabilité d'un jet qui heurte la cuvette et se reflète est plus importante.

La circuiterie de contrôle 302, 406 peut extraire ces propriétés (distance radiale, de vitesse radiale et dispersion) à l'aide d'algorithmes, notamment à l'aide d'analyse d'image et de comptage de pixels.

Différentes informations peuvent être extraites de telles cartes. Premièrement, une seul carte « distance-doppler » permet d'obtenir une propriété relative au jet d'urine, ce qui signifie qu'en une seule frame, le dispositif radar 100 peut distinguer le sexe de l'utilisateur en train d'uriner. La consommation de batterie est ainsi minimisée.

### Vitesse et Vmax

Un exemple d'algorithme consiste à déterminer une vitesse d'intérêt du front d'urine. Par exemple, cette vitesse d'intérêt est, ou est liée à, la vitesse positive la plus élevée (i.e., la vitesse radiale en direction du capteur radar 202) parmi les vitesses des fronts d'urine de la carte « distance-doppler ». Cette vitesse est appelée Vmax. Cette détermination peut se faire directement sur la carte « distance-doppler », notamment en identifiant le front d'urine (y compris de faible intensité) qui a la plus haute vitesse radiale. On rappelle que la carte « distance-doppler » donne déjà les vitesses radiales des fronts d'urine.

Alternativement, un exemple d'algorithme consiste à identifier une vitesse mesurée moyenne ou toute autre calcul à partir de la vitesse des fronts d'urine.

La propriété relative au jet d'urine est alors une vitesse radiale d'intérêt de fronts d'urine, comme une vitesse mesurée maximale.

### Distance radiale et Rmax

Un exemple d'algorithme consiste à déterminer la distance maximale entre le capteur radar 202 et l'ensemble des fronts d'urine. A cet égard, l'algorithme peut identifier le front d'urine avec la vitesse positive la plus élevée (i.e. la vitesse radiale en direction du capteur radar, appelée Vmax) puis récupérer la position radiale dudit front d'urine. Cette distance radiale est appelée Rmax.

Le front d'urine en sortie de l'urètre, ou à proximité, est considéré comme étant le plus rapide car la composante radiale est la plus importante.

La propriété relative au jet d'urine est alors une distance radiale d'intérêt entre le capteur radar 202 et la sortie de l'urètre.

### Niveau de réflexion et Npix

Un exemple d'algorithme consiste à identifier un niveau de réflexion des ondes radars. Ce niveau de réflexion est appelé Npix et il dépend de la dispersion du jet d'urine. A cet égard, l'algorithme peut compter le nombre de fronts ou le nombre de fronts ayant une intensité au-dessus d'un seuil prédéterminé. Concrètement, cela revient à mesurer l'aire des zones non uniformes sur la carte « distance doppler », indépendamment de l'intensité de l'objets dans chaque zone (la couleur).

La propriété relative au jet d'urine est alors un niveau de dispersion du jet d'urine (soit lors du jet d'urine lui-même, soit par les réflexions du jet d'urine sur la cuvette).

### Utilisation des propriétés relatives au jet d'urine

Dans un mode de réalisation, les propriétés relatives au jet d'urine permettent d'obtenir des informations physiologiques de l'utilisateur. Par exemple, la vitesse d'intérêt peut être corrélée à la pression de la vessie, aux pertes de charges ou au débit, qui peuvent eux-mêmes être corrélés à des pathologies (hypertrophie, cancer, trouble nerveux, etc.). Par exemple, le niveau de réflexion peut être corrélée au caractère laminaire ou turbulent du jet d'urine.

Dans un mode de réalisation, les propriétés relatives au jet d'urine sont utilisées pour identifier l'auteur du jet d'urine c'est-à-dire la personne en train d'uriner. En particulier, l'identification peut comprendre la discrimination entre homme et femme.

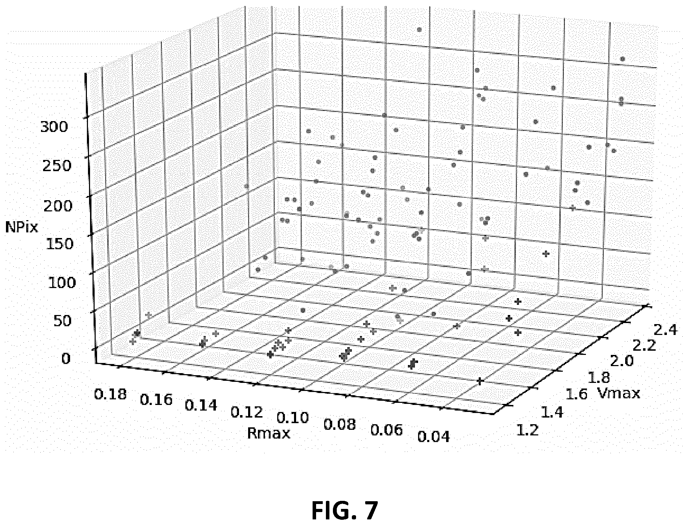

La figure 7 illustre une représentation tridimensionnelle d'une pluralité d'utilisateurs : chaque point représente une miction par un utilisateur, chaque point étant placé par sa valeur de Npix, Rmax et Vmax. Les points associés à des utilisateurs homme sont représentés par des croix et les points associés à des utilisateurs femme sont représentés par des ronds.

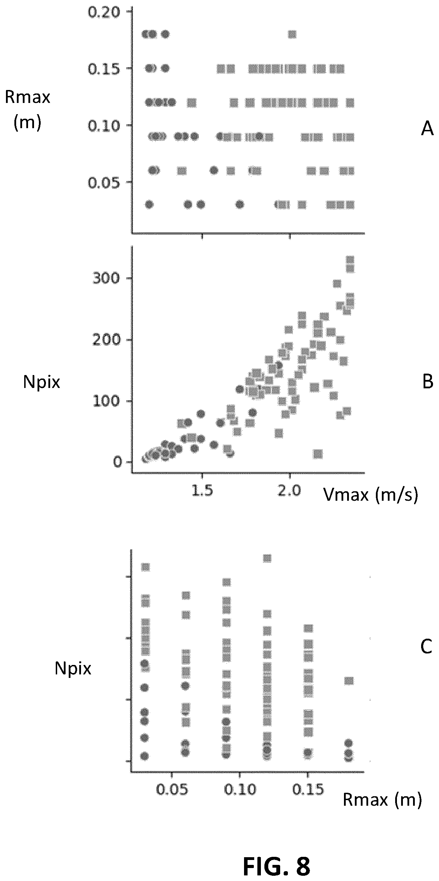

La figure 8 illustre des projections bidimensionnelles de la représentation tridimensionnelle précitées (avec un autre ensemble de données). Dit autrement, on a une représentation des trois propriétés décrites précédemment (Vmax, Rmax, Npix) en fonction chacun des unes des autres. La figure 8 illustre trois graphes A, B, C. Le graphe A illustre Rmax (en m) en fonction de Vmax (en m/s) ; le graphe B illustre Npix (en nombre) en fonction de Vmax (en m/s) ; le graphe C illustre Npix (en nombre) en fonction de Rmax (en m).

Ces représentations permettent de mettre visuellement en évidence des preuves de concept de discrimination du sexe à l'aide d'au moins une propriété obtenue par le capteur radar. Les carrés représentent des utilisateurs femme et les ronds des utilisateurs homme. Les données des figure 8 ont été obtenues sur une campagne interne avec 5 hommes et 5 femmes, avec une dizaine de mictions chacun au moins.

Les utilisateurs homme et les utilisateurs femme sont nettement identifiables sur certaines projections illustrées.

Par exemple, un apprentissage sur un ensemble de données labelées permet de déterminer des clusters homme/femme. Par exemple, dans les représentations bidimensionnelles, l'apprentissage peut déterminer une fonction de classification F(Rmax ;Vmax) qui partitionne l'espace et permet de clusteriser (ou partitionner) homme et femme. Les clusters homme/femme du graphe C de la figure 8 peuvent être séparées par une fonction de classification F(NPix ; Vmax) parce que les femmes ont généralement une Vmax supérieure aux hommes. Par exemple, la fonction de classification A.NPix + B.Vmax + C = 0 définit une droite qui sépare l'espace des couples (NPix ; Vmax) en deux. Un nouveau couple de point (NPix' ; Vmax') sera classifié en homme ou femme en fonction de la valeur de A.NPix'+BVmax', qui sera soit inférieure à 0 ou supérieure à 0. Des fonctions de classification F plus précises peuvent être définies pour définir des clusters plus restreints. Plus généralement, on parle de règles de classification, obtenues à l'aide de données labellisées.

Une classification à l'aide des propriétés NPix et Vmax donne des résultats de classification exploitable.

Une classification à l'aide des trois propriétés, NPix, Vmax et Rmax peut permettre d'affiner encore les résultats. Dans ce cas, on définit une fonction de classification F(NPix ; Vmax ; Rmax) qui compartimente l'espace des trouples (NPix ; Vmax ; Rmax).

Grâce à un tel algorithme, aucune calibration n'est nécessaire sur l'utilisateur.

Le calcul des propriétés du jet d'urine peut être effectué par la circuiterie de contrôle 302, 406. L'attribution du jet d'urine à une utilisateur particulier peut être effectuée par la circuiterie de contrôle. L'exécution en locale permet de rapidement identifier l'utilisateur.

### Exemple d'explications physiques

Chez une femme, le jet est rapidement turbulent et plus éloigné du capteur radar, ce qui crée davantage de front d'urine donc une plus grande réflexion du signal radar, d'où un NPix généralement plus élevée.

La distance radiale Rmax est plus faible chez un homme du fait de la position de l'urètre chez l'homme plus du capteur radar 202 que chez la femme. Néanmoins, lors des résultats présents, certains urètres étaient hors du FoV du capteur radar, rendant ainsi plus délicate l'utilisation de Rmax pour classifier. En revanche, Npix et Vmax permettent dans ce cas de classifier.

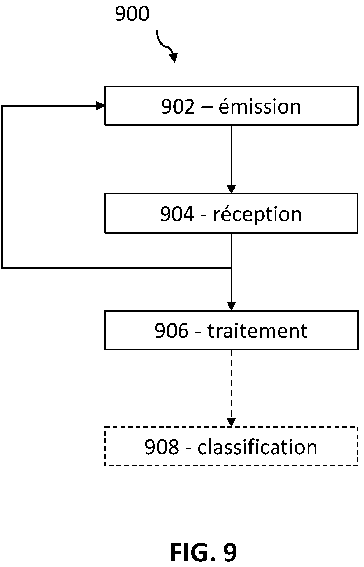

La figure 9 illustre un diagramme 900 représentant les étapes d'une méthode de mesure. Les étapes de la méthode 900 peuvent être mises en œuvre par la circuiterie de contrôle 302, 406. Dans une étape 902, la circuiterie de contrôle pilote le capteur radar pour émettre des signaux. Dans une étape 904, la circuiterie de contrôle pilote le capteur radar pour recevoir des signaux réfléchis. Les étapes 902, 904 peuvent être répétées plusieurs fois pour créer une image. Dans une étape 906, la circuiterie de contrôle traite les signaux réfléchis. Des exemples de traitements ont été décrits précédemment. En particulier, la circuiterie de contrôle calcule au moins une propriété du jet d'urine parmi notamment la vitesse (par exemple Vmax), la distance radiale (par exemple Rmax), un niveau de dispersion (par exemple Npix). Dans une étape 908, la circuiterie de contrôle utilise une ou plusieurs règles de classification stockées dans la circuiterie de contrôle pour classifier le jet d'urine comme venant d'un homme ou d'une femme et ainsi identifier l'utilisateur. En particulier, la circuiterie de contrôle 302, 406 classifie le jet d'urine à l'aide de l'au moins une propriété du jet d'urine. Par identification, il est signifié que la circuiterie de contrôle peut attribuer le jet d'urine à un profil utilisateur donné parmi une pluralité de profils utilisateur stockés dans la circuiterie de contrôle 302, 406. En particulier, l'attribution peut se faire à un profil homme ou à un profil femme en fonction de la classification. Dans ce mode de réalisation, la circuiterie de contrôle stocke un profil de chaque sexe au maximum. Dans un mode de réalisation, la circuiterie de contrôle stocke au plus un profil de chaque sexe.

En fonction de la classification de l'étape 908, la circuiterie de contrôle peut mettre en oeuvre ou non un procédé d'analyse d'urine tel que décrit dans les documents de brevet précités. Par exemple, si l'étape de classification 908 détermine que le jet d'urine est attribué à un profil femme, une analyse d'urine peut être mise en oeuvre pour déterminer un taux d'hormone lié au cycle menstruel. Inversement, si l'étape de classification 908 détermine que le jet d'urine est attribué à un profil homme, aucune analyse d'urine n'est lancée. Cela permet de drastiquement économiser des sessions d'analyse d'urine en identifiant correctement la personne en train d'urine.

L'étape de classification 908 peut être effectuée par le smartphone ou le serveur et non pas par la circuiterie de contrôle. De la même façon, une partie de l'étape de traitement 906 peut être effectuée par le smartphone ou le serveur. Comme la détection de l'utilisateur peut conditionner le déclenchement d'une mesure et qu'une miction ne dure que quelques secondes, il peut être important que l'étape de classification soit effectuée par la circuiterie de contrôle du dispositif radar lui-même. En effet, un aller-retour avec le serveur nécessite une connexion internet stable et une disponibilité immédiate du serveur. Pour avoir un algorithme embarqué, du clustering sans *machine learning* peut être utilisé.

### Détecteur d'urine

Afin de déclencher une acquisition radar uniquement lorsqu'un jet d'urine est en cours (pour économiser de la batterie), les toilettes peuvent intégrer un détecteur d'urine 210. Plus particulièrement, le détecteur d'urine est monté dans le dispositif radar 100. Le détecteur d'urine 210 peut détecter la présence d'un jet d'urine. Le détecteur d'urine 210 peut comprendre un capteur de température 220 monté dans le boitier 200, par exemple au niveau de l'orifice de collecte 204. Lorsque de l'urine à plus de 35°C ruisselle sur le boitier, le capteur de température 220 va relever une augmentation soudaine de température.

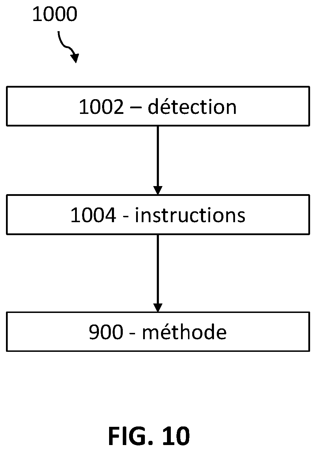

La figure 10 illustre un diagramme 1000 représentant une méthode d'activation du capteur radar 202. Dans une étape 1002, le détecteur d'urine détecte la présence d'un jet d'urine. Dans une étape 1004, en réponse à ladite détection, la circuiterie de contrôle 302, 406 commande le capteur radar 202 d'effectuer la méthode 900.

Pour identifier un paramètre du jet d'urine, il n'est pas toujours nécessaire d'obtenir une image radar de l'intégralité de la miction. En particulier, à des fins d'identification sur la base des propriétés du jet d'urine précités, une seule image radar du jet d'urine peut suffire. Manquer le début de la miction ne pose donc pas de difficultés particulières. La méthode présentée est donc particulièrement robuste à la temporalité d'activation du capteur radar.

Dans un autre mode de réalisation, le capteur d'urine est remplacé par un capteur de présence d'utilisateur. Ce capteur peut être une cellule de charge ou un capteur optique. Néanmoins, un tel capteur de présence ne peut pas informer le capteur radar 202 qu'un jet d'urine est en cours mais simplement qu'un utilisateur est assis. Par conséquent, il peut être prévu que le capteur radar envoie des ondes prospectives quelques secondes avant que la miction ne survienne pour être certain d'acquérir des signaux radars réfléchis par le jet d'urine.

### Positionnement du capteur radar dans les toilettes

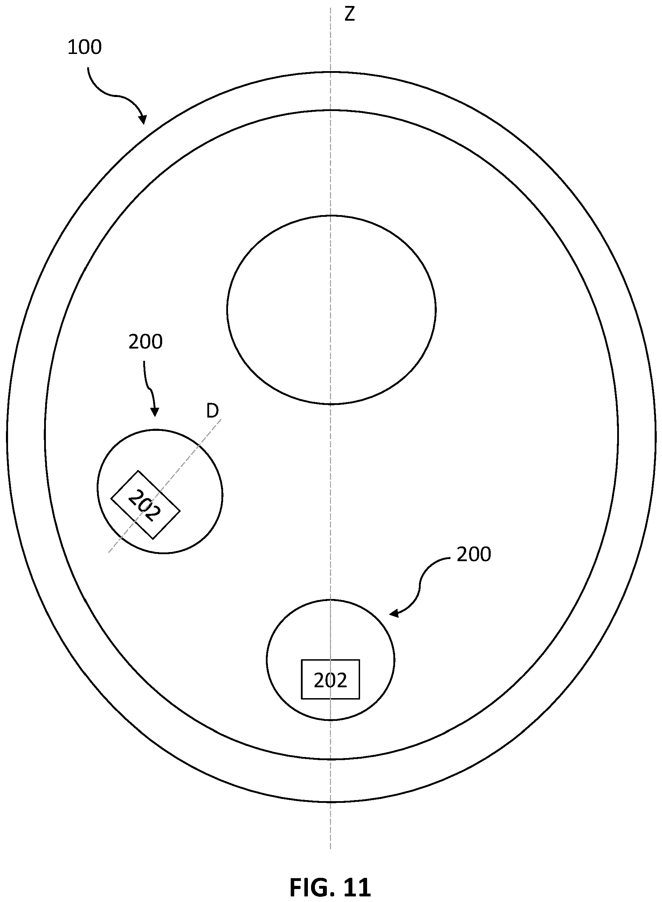

La figure 11 illustre différents emplacements possibles pour le dispositif radar 100 et notamment le module radar 202 à l'intérieur du boitier.

Dans un mode de réalisation, le capteur radar 202 est centré par rapport à un axe de symétrie D du boitier 200 ; si l'utilisateur centre le boitier 200 sur un axe de symétrie Z des toilettes, alors le capteur radar 200 est centré par rapport aux toilettes. Ce positionnement central permet d'observer des vitesses radiales plus proches de la vitesse réelle d'éjection. En contrepartie, le risque de ne pas voir l'intégralité du jet augmente.

Dans un mode de réalisation, le capteur radar 202 est décentré par rapport à un axe de symétrie du boitier 200 ; si l'utilisateur centre le boitier 200 sur un axe de symétrie Z des toilettes, alors le capteur radar 200 est légèrement décentré par rapport aux toilettes. Ce positionnement permet au capteur radar 202 d'observer le jet légèrement de côté (dans l'hypothèse où en moyenne les jets d'urine se font dans l'axe de symétrie) et donc d'accroitre la probabilité d'observer l'intégralité du jet et d'augmenter la précision de la mesure.

Dans un mode de réalisation, le boitier 200 est positionné à distance de l'axe de symétrie des toilettes, c'est-à-dire qu'il est légèrement décalé à droite ou à gauche

Dans le cas d'une intégration du dispositif radar à un dispositif d'analyse d'urine, le boitier 200 doit se trouver sous le jet d'urine, ce qui impose un positionnement du boitier dans la partie proximale de la cuvette et préférablement à proximité d'un axe de symétrie Z des toilettes.

### Intégration d'un capteur radar dans un dispositif d'analyse d'urine

Dans un mode de réalisation, le dispositif radar est intégré à un dispositif d'analyse d'urine. Le dispositif d'urine a été décrit dans les documents WO2021/175909, WO2021/175944 (numéro de publication), FR2109383, FR2109384, FR2109391, and FR2109392 (numéro de dépôt). La classification d'un utilisateur peut être une étape préalable au déclenchement d'une analyse d'urine. La classification de l'utilisateur permet aussi d'attribuer les résultats de l'analyse au bon profil.

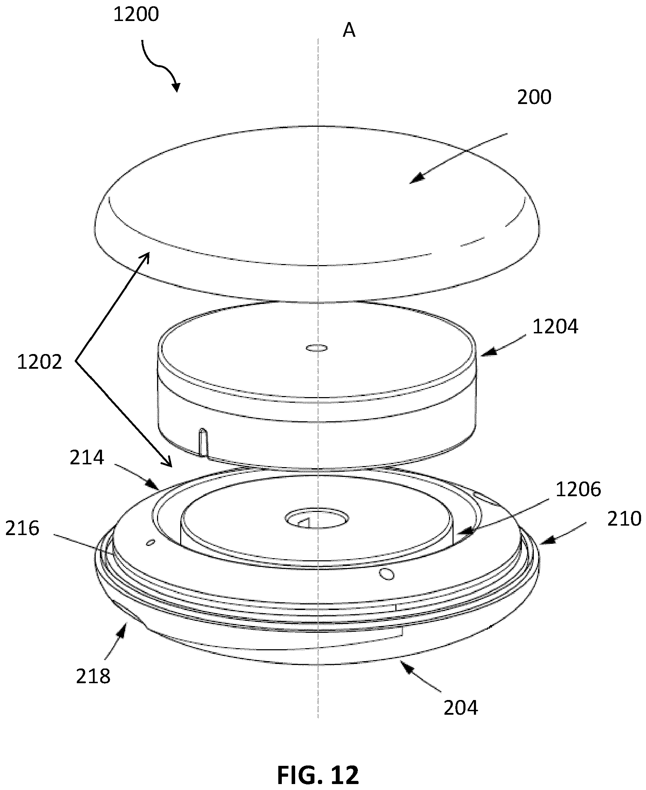

Comme illustré en détail sur la figure 12, le dispositif d'analyse urine 1200 comprend une station 1202 et une cartouche 1204, montée amovible dans la station 1202. La station 1202 comprend notamment le boitier 200 qui est, selon une réalisation particulière, formé comme un assemblage de deux demi-coques. Le boitier 200 renferme un ensemble de test. L'ensemble de test est destiné à analyser l'urine étant reçue dans le dispositif d'analyse d'urine 100. La station 1202 comporte en outre un logement annulaire 1206, à l'intérieur du boitier 200, agencé autour d'un axe de rotation A. Le logement annulaire 1206 est configuré pour recevoir au moins partiellement la cartouche 1204 montée à rotation autour de l'axe de rotation A (une fois en position dans le logement annulaire 212). La cartouche 1204 comprend une pluralité de supports de test intégrant un réactif, par exemple un réactif sec agencés le long d'un cercle ou d'un arc de cercle autour de l'axe de rotation A. Dans un mode de réalisation et pour la suite de la description, les supports de tests sont des bandelettes de test. Les tests de support sont enfermés individuellement dans une chambre

Le logement annulaire 1206 s'étend typiquement sur 360° et forme une gorge configurée pour recevoir en partie la cartouche 1204.

La station 1202 comprend en outre l'orifice de collecte 218, positionnée par exemple sur la coque arrière sur la figure 12. L'orifice de collecte 218 peut recevoir l'urine ruisselant par gravité sur la surface extérieure du boitier 204. Un orifice de vidange (non illustré) est aussi inclus pour évacuer le liquide du dispositif 1200.

Dans un mode de réalisation, le boitier 200 a un diamètre, mesuré dans la direction normale à l'axe A, compris entre 50 mm et 150 mm, par exemple voisin de 100 mm.

L'ensemble de test comprend une pompe, un injecteur et un analyser, non visible sur la figure 12. La pompe aspire l'urine depuis l'orifice de collecte 218 et ensuite l'injecteur injecte l'urine sur un support de test de la cartouche, puis l'analyseur obtient des propriétés du support de test après qu'il a contacté l'urine. L'injecteur et la cartouche peuvent bouger l'un par rapport à l'autre de sorte que l'injecteur puisse percer la chambre.

## Revendications

1. Méthode de mesure relative à un jet d'urine d'un utilisateur en cours de miction pour identifier un utilisateur de toilettes, la méthode de mesure utilisant un capteur radar (202) et comprenant au moins les étapes suivantes :
- l'émission par le capteur radar (202) d'au moins un signal radar, en direction du jet d'urine,
- la réception par le capteur radar (202) d'un signal radar réfléchi, le signal radar reçu comprenant des réflexions du signal émis, les réflexions étant provoquées par au moins le jet d'urine,
- le traitement du signal radar reçu pour déterminer au moins une propriété relative au jet d'urine, et
- l'attribution du jet d'urine à un profil d'utilisateur parmi une pluralité de profils d'utilisateur à l'aide de l'au moins une propriété du jet d'urine.

2. Méthode de mesure selon la revendication 1, dans laquelle la propriété relative au jet d'urine comprend au moins une distance d'intérêt entre le capteur radar (202) et le jet d'urine, par exemple la distance entre l'origine du jet d'urine et le capteur radar (202).

3. Méthode de mesure selon la revendication 2, dans lequel ladite distance d'intérêt est obtenue par :
- l'identification d'une vitesse radiale d'intérêt liée à la vitesse radiale maximale (Vmax) d'un front d'urine du jet d'urine,
- l'obtention de la distance radiale (Rmax) correspondant à cette vitesse d'intérêt, ladite distance radiale (Rmax) correspondant à la distance d'intérêt.

4. Méthode de mesure selon l'une quelconque des revendications 1 à 3, dans laquelle la propriété relative au jet d'urine comprend un niveau de dispersion (NPix) du jet d'urine, par exemple ledit niveau de dispersion est obtenu par le calcul d'un niveau de réflexion des signaux radars réfléchis.

5. Méthode de mesure selon l'une quelconque des revendications 1 à 4, dans laquelle la propriété du jet d'urine comprend au moins une vitesse d'intérêt d'un front d'urine du jet d'urine.

6. Méthode de mesure selon la revendication 5, dans laquelle la vitesse d'intérêt du jet d'urine comprend la vitesse mesurée maximale (Vmax) du jet d'urine.

7. Méthode de mesure selon l'une quelconque des revendications 1 à 6, dans laquelle l'attribution comprend une attribution entre un profil d'utilisateur associé à un homme et un profil d'utilisateur associé à une femme.

8. Méthode de mesure selon la revendication 6 ou 7, dans laquelle l'attribution se fait par classification sur la base d'au moins une propriété relative au jet d'urine et d'une fonction de classification.

9. Méthode de mesure selon l'une quelconque des revendications 1 à 8, dans laquelle le capteur radar (202) fonctionne par frame, chaque frame étant générée par une pluralité de chirps, et les étapes d'émission et de réception étant mises en oeuvre pour chaque chirp.

10. Méthode de mesure selon l'une quelconque des revendications 1 à 9, dans laquelle le capteur radar (202) est un capteur radar de type « *Frequency Modulated Continuous Wave* », FMCW, ou le signal radar est FMCW.

11. Méthode de mesure selon l'une des revendications 1 à 10, dans laquelle le traitement du signal comprend un calcul d'au moins une réponse « range-doppler ».

12. Méthode de mesure selon l'une des revendications 1 à 11, comprenant une étape préalable, à l'aide d'un détecteur d'urine (210) de détermination de la présence d'un jet d'urine, la méthode comprenant en réponse à ladite détection, une étape d'activation du capteur radar (202).

13. Programme d'ordinateur comprenant des instructions aptes à mettre en oeuvre une méthode selon l'une quelconque des revendications 1 à 12 lorsque les instructions sont exécutées par un processeur du capteur radar selon la revendication 1.

14. Dispositif radar (100) comprenant :
- un boîtier (200), apte à être positionné sur une paroi interne d'une cuvette (106) de toilette,
- un capteur radar (202), logé dans le boitier (200), et apte à émettre des ondes radar en direction de l'ouverture de la cuvette de toilette, le capteur radar (202) étant apte à mettre en oeuvre la méthode selon l'une quelconque des revendications 1 à 13.

15. Dispositif d'analyse d'urine (1500), comprenant :
- un dispositif radar (100) selon la revendication 14,
- un orifice de collecte (204) sur le boitier (200) pour recevoir de l'urine,
- un ensemble de test destiné à analyser l'urine reçue.

## Patentansprüche

1. Messverfahren in Bezug auf einen Urinstrahl eines urinierenden Benutzers, um einen Toilettenbenutzer zu identifizieren, wobei das Messverfahren einen Radarsensor (202) verwendet und mindestens die folgenden Schritte umfasst:
- das Aussenden mindestens eines Radarsignals durch den Radarsensor (202) in Richtung des Urinstrahls,
- Empfangen eines reflektierten Radarsignals durch den Radarsensor (202), wobei das empfangene Radarsignal Reflexionen des gesendeten Signals umfasst, wobei die Reflexionen durch mindestens den Urinstrahl verursacht werden,
- Verarbeiten des empfangenen Radarsignals, um mindestens eine Eigenschaft zu bestimmen, die sich auf den Urinstrahl bezieht, und
- Zuordnen des Urinstrahls zu einem Nutzerprofil aus einer Vielzahl von Nutzerprofilen anhand der mindestens einen Eigenschaft des Urinstrahls.

2. Messverfahren nach Anspruch 1, wobei die Eigenschaft bezüglich des Urinstrahls mindestens eine interessierende Entfernung zwischen dem Radarsensor (202) und dem Urinstrahl umfasst, z. B. die Entfernung zwischen dem Ursprung des Urinstrahls und dem Radarsensor (202).

3. Messverfahren nach Anspruch 2, wobei die interessierende Entfernung erhalten wird durch :
- Identifizieren einer interessierenden Radialgeschwindigkeit, die sich auf die maximale Radialgeschwindigkeit (Vmax) einer Urinfront des Urinstrahls bezieht,
- Ermitteln des radialen Abstands (Rmax), der dieser Geschwindigkeit von Interesse entspricht, wobei der radiale Abstand (Rmax) dem Abstand von Interesse entspricht.

4. Messverfahren nach einem der Ansprüche 1 bis 3, wobei die Eigenschaft bezüglich des Urinstrahls einen Streupegel (NPix) des Urinstrahls umfasst, z. B. wird der Streupegel durch die Berechnung eines Reflexionspegels der reflektierten Radarsignale erhalten.

5. Messverfahren nach einem der Ansprüche 1 bis 4, wobei die Eigenschaft des Urinstrahls mindestens eine interessierende Geschwindigkeit einer Urinfront des Urinstrahls umfasst.

6. Messverfahren nach Anspruch 5, wobei die interessierende Geschwindigkeit des Urinstrahls die maximale gemessene Geschwindigkeit (Vmax) des Urinstrahls umfasst.

7. Messverfahren nach einem der Ansprüche 1 bis 6, wobei die Zuordnung eine Zuordnung zwischen einem Nutzerprofil, das einem Mann zugeordnet ist, und einem Nutzerprofil, das einer Frau zugeordnet ist, umfasst.

8. Messverfahren nach Anspruch 6 oder 7, wobei die Zuordnung durch Klassifizierung auf der Grundlage von mindestens einer Eigenschaft in Bezug auf den Urinstrahl und einer Klassifizierungsfunktion erfolgt.

9. Messverfahren nach einem der Ansprüche 1 bis 8, wobei der Radarsensor (202) frameweise arbeitet, wobei jeder Frame durch eine Vielzahl von Chirps erzeugt wird und die Schritte des Sendens und Empfangens für jeden Chirp durchgeführt werden.

10. Messverfahren nach einem der Ansprüche 1 bis 9, wobei der Radarsensor (202) ein Radarsensor vom Typ "Frequency Modulated Continuous Wave", FMCW, ist oder das Radarsignal FMCW ist.

11. Messverfahren nach einem der Ansprüche 1 bis 10, wobei die Signalverarbeitung eine Berechnung von mindestens einer "Range-Doppler"-Antwort umfasst.

12. Messverfahren nach einem der Ansprüche 1 bis 11, umfassend einen vorherigen Schritt, bei dem mithilfe eines Urindetektors (210) das Vorhandensein eines Urinstrahls bestimmt wird, wobei das Verfahren als Reaktion auf die Detektion einen Schritt umfasst, bei dem der Radarsensor (202) aktiviert wird.

13. Computerprogramm mit Anweisungen, die geeignet sind, ein Verfahren nach einem der Ansprüche 1 bis 12 zu implementieren, wenn die Anweisungen von einem Prozessor des Radarsensors nach Anspruch 1 ausgeführt werden.

14. Radarvorrichtung (100) mit :
- einem Gehäuse (200), das geeignet ist, an einer Innenwand eines Toilettenbeckens (106) positioniert zu werden,
- einen Radarsensor (202), der in dem Gehäuse (200) untergebracht ist und Radarwellen in Richtung der Öffnung der Toilettenschüssel aussenden kann, wobei der Radarsensor (202) das Verfahren nach einem der Ansprüche 1 bis 13 ausführen kann.

15. Vorrichtung zur Untersuchung von Urin (1500), umfassend :
- einer Radarvorrichtung (100) nach Anspruch 14,
- eine Sammelöffnung (204) an dem Gehäuse (200) zur Aufnahme von Urin,
- eine Testanordnung zum Analysieren des aufgenommenen Urins.

## Claims

1. A measurement method of a stream of urine from a user during urination to identify a toilet user, the measuring method using a radar sensor (202) and comprising at least the following steps:
- emission by the radar sensor (202) of at least one radar signal, in the direction of the urine stream,
- reception by the radar sensor (202) of a reflected radar signal, the received radar signal comprising reflections of the transmitted signal, the reflections being caused by at least the urine stream,
- processing the received radar signal to determine at least one property relating to the urine stream, and
- assigning the urine stream to one of a plurality of user profiles on the basis of the at least one property of the urine stream.

2. The measurement method according to claim 1, wherein the urine stream property comprises at least one distance of interest between the radar sensor (202) and the urine stream, for example the distance between the origin of the urine stream and the radar sensor (202).

3. The measurement method according to claim 2, wherein said distance of interest is obtained by :
- identifying a radial velocity of interest related to the maximum radial velocity (Vmax) of a urine front of the urine stream,
- obtaining the radial distance (Rmax) corresponding to this velocity of interest, said radial distance (Rmax) corresponding to the distance of interest.

4. The measurement method according to any one of claims 1 to 3, wherein the property relating to the urine stream comprises a dispersion level (NPix) of the urine stream, for example said dispersion level is obtained by calculating a reflection level of the reflected radar signals.

5. The measurement method according to any one of claims 1 to 4, wherein the urine stream property comprises at least one velocity of interest of a urine front of the urine stream.

6. The measurement method according to claim 5, wherein the velocity of interest of the urine stream comprises the maximum measured velocity (Vmax) of the urine stream.

7. The measurement method according to any one of claims 1 to 6, wherein the assignment comprises an assignment between a user profile associated with a male and a user profile associated with a female.

8. The measurement method according to claim 6 or 7, wherein the allocation is by classification on the basis of at least one property relating to the urine stream and a classification function.

9. The measurement method according to any one of claims 1 to 8, wherein the radar sensor (202) operates per frame, each frame being generated by a plurality of chirps, and the transmission and reception steps being implemented for each chirp.

10. The measurement method according to any one of claims 1 to 9, wherein the radar sensor (202) is a "Frequency Modulated Continuous Wave", FMCW, radar sensor, or the radar signal is FMCW.

11. The measurement method according to one of claims 1 to 10, wherein signal processing comprises calculation of at least one range-doppler response.

12. The measurement method according to one of claims 1 to 11, comprising a prior step, using a urine detector (210), of determining the presence of a urine stream, the method comprising, in response to said detection, a step of activating the radar sensor (202).

13. A computer program comprising instructions adapted to implement a method according to any one of claims 1 to 12 when the instructions are executed by a processor of the radar sensor according to claim 1.

14. A radar device (100) comprising :
- a housing (200), suitable for positioning on an inner wall of a toilet bowl (106),
- a radar sensor (202), housed in the casing (200), and suitable for emitting radar waves in the direction of the toilet bowl opening, the radar sensor (202) being suitable for implementing the method according to any one of claims 1 to 13.

15. A urine analysis device (1500), comprising :
- a radar device (100) according to claim 14,
- a collection port (204) on the housing (200) for receiving urine,
- a test set for analyzing the urine received.
